## Europäisches Patentamt
## European Patent Office
## Office européen des brevets

(19)

(11) Veröffentlichungsnummer: **0 093 341**
**B1**

(12) **EUROPÄISCHE PATENTSCHRIFT**

(45) Veröffentlichungstag der Patentschrift:
**13.08.86**

(51) Int. Cl.⁴: **A 61 K 7/00, C 11 B 11/00**

(21) Anmeldenummer: **83103916.9**

(22) Anmeldetag: **21.04.83**

(54) **Wollwachs-Ersatzprodukt.**

(30) Priorität: **29.04.82 DE 3215912**

(43) Veröffentlichungstag der Anmeldung:
**09.11.83 Patentblatt 83/45**

(45) Bekanntmachung des Hinweises auf die Patenterteilung:
**13.08.86 Patentblatt 86/33**

(84) Benannte Vertragsstaaten:
**AT BE CH DE FR GB IT LI NL SE**

(73) Patentinhaber: **Henkel Kommanditgesellschaft auf Aktien, Postfach 1100 Henkelstrasse 67, D-4000 Düsseldorf- Holthausen (DE)**

(72) Erfinder: **Scheuffgen, Ingeborg, Spulgasse 3, D-4040 Neuss (DE)**

(56) Entgegenhaltungen:
FETTE, SEIFEN, ANSTRICHMITTEL, Band 84, Nr. 3, März 1982, Seiten 126-129, Münster, DE. H. HERMSDORF: "Eigenschaften und Einsatzmöglichkeiten eines neuen Wollwachs-Substituts"
H. JANISTYN: "Handbuch der Kosmetika und Riechstoffe", 3. Auflage, Band 1, "Die kosmetischen Grundstoffe", Seiten 227,414,436,437,909,910, Dr. Alfred Hüthig Verlag GmbH, Heidelberg, DE.

**Beschreibung**

Gegenstand der Erfindung ist ein neuartiges Ersatzprodukt für Wollwachs, bestehend aus einer Mischung synthetischer, grenzflächenaktiver Fettfolgeprodukte und dessen Verwendung zur Herstellung kosmetischer und pharmazeutischer Salben, Cremes, Emulsionen und Stiftpräparate.

Wollwachs ist ein bekanntes Hilfsmittel zur Herstellung kosmetischer Zubereitungen. Es dient sowohl in der raffinierten und entsäuerten Form als auch in Form verschiedener Derivate zur Herstellung von Emulsionen und Stiftpräparaten. Seine Zusammensetzung ist sehr komplex und nur teilweise aufgeklärt. Zu einem großen Teil besteht es aus Estern langkettiger Carbonsäuren und Hydroxycarbonsäuren mit Cholesterin- und Lanosterinalkohol. Wollwachs wird besonders wegen seiner hohen Wasseraufnahme als Emulgierhilfsmittel, Stabilisator und Co-Emulgator sowie wegen seiner hautweichmachenden Eigenschaften als "Emollient" in Salben und Stiftpräparaten geschätzt. Trotz der genannten Vorzüge weist das Wollwachs aber auch gewisse Nachteile auf. So wird über Allergien berichtet, die durch Wollwachs enthaltende Kosmetika ausgelöst wurden und die von Pestizidrückständen und gewissen polycyclischen Alkoholen in dem Naturprodukt herrühren sollen. Aus diesem Grunde ist in Deutschland gemäß Kosmetikverordnung von 1977 ein Hinweis auf den Lanolingehalt auf der Verpackung vorgeschrieben. Ein weiterer Nachteil des Naturproduktes ist sein merklicher Eigengeruch, der eine stärkere Parfümierung bedingt, die ebenfalls von Personen mit empfindlicher Haut oder Neigung zu Allergien schlecht vertragen wird. Darüber hinaus unterliegt Wollwachs als Naturprodukt erheblichen Schwankungen in Qualität, Preis und Verfügbarkeit, so daß allein aus diesen Gründen ein synthetisches Ersatzprodukt erwünscht ist. Aufgrund der komplexen Zusammensetzung des Wollwachses ist eine chemische Synthese nicht möglich. Bisherige Versuche, anwendungstechnisch vergleichbare Alternativen zu schaffen, haben zu wenig befriedigenden Ersatzprodukten geführt, die weder hinsichtlich der emulsionstechnischen noch der hautweichmachenden Eigenschaften dem Naturprodukt entsprechen. Es bestand daher die Aufgabe, ein synthetisches Ersatzprodukt für Wollwachs zu schaffen, das dem Naturprodukt sowohl in der Konsistenz als auch in den anwendungstechnischen und kosmetischen Eigenschaften möglichst nahe kommt.

Die Aufgabe wurde erfindungsgemäß gelöst durch eine Kombination von an sich bekannten, synthetischen, grenzflächenaktiven Fettfolgeprodukten, die innerhalb verhältnismäßig eng begrenzter Mengenverhähnisse die erwünschten wollwachsähnlichen Eigenschaften, insbesondere eine vergleichbare Konsistenz, eine vergleichbare stabilisierende Wirkung auf kosmetische Emulsionen und Cremes und ein ähnlich günstiges Hautgefühl der damit hergestellten Präparate aufweist. Die erfindungsgemäße Kombination ist gekennzeichnet durch einen Gehalt von

40—60 Gew.-% eines Mischesters aus äquimolaren Mengen eines Pentaerythrit-di-fettsäureesters und eines Zitronensäure-di-fettalkoholesters

25—45 Gew.-% Ölsäure-mono/diglycerid

3—15 Gew.-% Palmitin- und/oder Stearinsäuremono/diglycerid

3—10 Gew.-% Anlagerungsprodukt von 3—7 Mol Ethylenoxid an ein pflanzliches Sterin, wobei die Summe der Bestandteile jeweils 100 Gew.-% betragen muß.

Die in der erfindungsgemäßen Kombination einzusetzenden Mischester aus äquimolaren Mengen Pentaerythrit-di-fettsäureester und Zitronensäure-di-fettalkoholester sind bekannt aus der deutschen Patentschrift 1.165.574. Von den in dieser Druckschrift genannten Produkten ist für die Herstellung des erfindungsgemäßen Wollwachs-Ersatzproduktes der dort im Beispiel 1 beschriebene Mischester aus einem Pentaerythrit-di-kokosfettsäureester und einem Zitronensäure-di-octadecylester besonders gut geeignet.

Das in dem erfindungsgemäßen Wollwachs-Ersatzprodukt einzusetzende Ölsäure-mono-di-glycerid ist ein Produkt, welches z.B. durch Veresterung von Glycerin mit 1—2 Mol eines Oleins, also einer Fettsäure, die überwiegend aus Ölsäure besteht, hergestellt werden kann. Solche Produkte setzen sich im allgemeinen aus Mono-, Di- und Triestern zusammen, wobei der Monoester-Gehalt mindestens 40 Gew.-% ausmachen sollte.

Das in den erfindungsgemäßen Produkten einzusetzende Palmitin- und/oder Stearinsäure-monodiglycerid ist ein Produkt, welches z.B. durch Veresterung von Glycerin mit 1—2 Mol eines technischen Stearins, also einer Fettsäure, die vorwiegend aus Palmitinsäure und Stearinsäure besteht, hergestellt werden kann. Solche Produkte setzen sich ebenfalls aus Mono-, Di- und Triestern zusammen, der Monoester-Gehalt sollte jedoch mindestens ca. 40 Gew.-% ausmachen.

Das in dem erfindungsgemäßen Wollwachs-Ersatzprodukt zu verwendende Anlagerungsprodukt von Ethylenoxid an ein pflanzliches Sterin wird durch Umsetzung des Sterins mit 3—7 Mol Ethylenoxid in Gegenwart eines vorzugsweise basischen Katalysators, z.B. von Kaliumhydroxid oder Natriummethylat unter Druck hergestellt. Als pflanzliches Sterin eignet sich z.B. das aus dem unverseifbaren Anteil pflanzlicher Öle isolierte und raffinierte Steringemisch. Hierbei handelt es sich um Mischungen verschiedener Sterinverbindungen mit geringeren Anteilen unbekannter Begleitstoffe pflanzlichen Ursprungs. Definierte Bestandteile solcher pflanzlicher Sterine sind zum Beispiel Sitosterin, Campesterin, Stigmasterin, Brassicasterin, α-Spinasterin, Sargasterin und Focusterin in unterschiedlichen Mengenverhältnissen. Bevorzugt geeignet ist das Anlagerungsprodukt von ca. 5 Mol Ethylenoxid an das raffinierte Sojasterin.

2

Ganz besonders gut geeignet und universell als Wollwachs-Ersatzprodukt einsetzbar ist eine Kombination aus:

50 Gew.-% des Mischesters aus äquimolaren Mengen von Pentaerythrit-di-kokosfettsäureester und Zitronensäure-d-n-octadecylester
40 Gew.-% Ölsäuremono-diglycerid mit ca. 46 Gew.-% Monoglyceridgehalt
5 Gew.-% Palmitin-stearinsäure-mono-diglycerid mit ca. 46 Gew.-% Monoglyceridgehalt
5 Gew.-% des Anlagerungsproduktes von 5 Mol Ethylenoxid an raffiniertes Sojasterin.

Diese bevorzugte Kombination weist eine dem natürlichen Wollwachs ähnliche Konsistenz auf und liegt bezüglich der technischen Kenndaten recht nahe dem natürlichen Produkt. Vor allem aber verhält sie sich in typischen Anwendungsrezepturen dem natürlichen Wollwachs verblüffend ähnlich. So zeigen die damit hergestellten Cremes, Salben und Lotionen, gleichgültig ob sie dem Wasser-in-Öl oder dem Öl-in-Wasser (O/W)-Typ angehören, ein ähnliches Aussehen in Bezug auf Feinteiligkeit, Glätte, Geschmeidigkeit, Glanz bzw. Transparenz wie die im Vergleich dazu mit natürlichem Wollwachs hergestellten Präparate. Darüber hinaus liegen die Präparate in einem sehr ähnlichen Viskositätsbereich und zeigen praktisch keine Unterschiede in der Lagerstabilität, wenn man zum Vergleich entsprechende Rezepturen heranzieht, die unter Verwendung von Wollwachs hergestellt wurden.

Die erfindungsgemäßen Wollwachs-Ersatzprodukte besitzen darüber hinaus den Vorteil, daß ihre Verwendung in kosmetischen und pharmazeutischen Präparaten zu Produkten führt, die auch von Personen vertragen werden, die gegenüber Wollwachs allergisch reagieren. Da die neuen Wollfett-Ersatzprodukte weitgehend geruchlos sind, ermöglichen sie eine sparsame Parfümierung der damit hergestellten Präparate und tragen auf diese Weise ebenfalls zur Verbesserung der Hautverträglichkeit kosmetischer Präparate bei.

Die neuen Wollwachs-Ersatzprodukte sind mit den üblichen sonstigen Bestandteilen kosmetischer Salben, Cremes, Lotionen und Stiftpräparate verträglich, sie können daher mit allen bekannten Hilfsstoffen problemlos kombiniert werden. Solche Hilfsstoffe sind z.B. anionische und nichtionogene Emulgatoren oder Salbengrundlagen, Seifen und kosmetische Fettstoffe wie z.B. Vaseline, Paraffinöle, Hartparaffine, Mikrowachse, Triglyceridöle, Wachse (z.B. Walrat, Bienenwachs), Fettalkohole, Fettsäuren, Fettsäureester, Glycerin, Parfümöle, Konservierungsstoffe usw.

Die nachfolgenden Beispiele sollen den Gegenstand der Erfindung näher erläutern ohne ihn jedoch hierauf zu beschränken:

Beispiele

1. Charakterisierung der in den Beispielen verwendeten Emulgator-Komponenten.

1.1 Mischester

Es wurde ein Mischester aus Zitronensäure-di-octadecylester und Pentaerythrit-di-kokosfettsäureester gemäß DE—PS 1.165.574, Beispiel 1c hergestellt. Das Umesterungsprodukt hatte folgende Kenndaten:

| | |
|---|---|
| Säurezahl | 0,8 |
| Verseifungszahl | 226 |
| Hydroxylzahl | 71 |

1.2 Ölsäure-mono-diglycerid

Das verwendete Produkt wurde aus einer technischen Ölsäure (Säurezahl 202, Jodzahl 92, Trübungspunkt 6°C) hergestellt und hatte folgende Kanndaten:

| | |
|---|---|
| Verseifungszahl | 177 |
| Jodzahl | 74 |
| Monoglyceridgehalt | 46% |
| freies Glycerin | ca. 1% |

1.3 Stearinsäure-mono-diglycerid

Das verwendete Produkt wurde aus einem technischen Stearin (1% $C_{12}$, 2% $C_{14}$, 45% $C_{16}$, 2% $C_{17}$, 47% $C_{18}$, Jodzahl 0,5) hergestellt und hatte folgende Kenndaten:

| | |
|---|---|
| Verseifungszahl | 171 |
| Steigschmelzpunkt | 58°C |
| Monoglyceridgehalt | 46% |
| freies Glycerin | ca. 1% |

1.4 Ethoxyliertes Sojasterin

Das verwendete Produkt wurde aus einem raffinierten Sojasterin durch Anlagerung von 5 Mol Ethylenoxid hergestellt und hatte folgende Kenndaten:

| | |
|---|---|
| Hydroxylzahl | ca. 100 |
| Schmelzbereich | 70—90°C |
| freies Sterin | ca. 10% (gaschromatographisch) |

2. Wollfett-Ersatzprodukt (erfindungsgemäß)

| | | Zum Vergleich: |
|---|---|---|
| Mischester nach 1.1: | 50 Gew.-% | natürliches Wollwachs |
| Ölsäure-mono-di-glycerid nach 1.2 | 40 Gew.-% | (wasserfrei) gemäß |
| Stearinsäure-mono-diglycerid nach 1.3 | 5 Gew.-% | Deutsches Arzneibuch, |
| Anlagerungsprodukt von 5 Mol Ethylenoxid an Sojasterin: | 5 Gew.-% | 8. Ausgabe (1978) |
| Technische Kenndaten: | | |
| Säurezahl | 3,8 | 0,8—2,0 |
| Verseifungszahl | 186 | 90—105 |
| Jodzahl | 33 | 18—32 |
| Hydroxylzahl | 151 | —— |
| Schmelzpunkt | 37°C | 36—42°C |

3. Prüfrezepturen
3.1 Flüssige Handlotion (O/W-Emulsion)

| | | |
|---|---|---|
| Wollwachs | 5 | — |
| Wollwachs-Ersatzprodukt (nach 2.) | — | 5 |
| O/W-Grundlage (Emulgade F$^{(R)}$)[1] | 2 | 2 |
| Stearin (50% $C_{16}$, 50% $C_{18}$) | 2 | 2 |
| Paraffinöl, dickflüssig | 2,5 | 2,5 |
| Decyloleat (Cetiol V$^{(R)}$) | 3 | 3 |
| Olivenöl | 1,5 | 1,5 |
| Triethanolamin | 1 | 1 |
| Wasser | 83,0 | 83,0 |
| Emulgierbarkeit | gut | gut |
| Struktur, Aussehen nach Herstellung | glatt, glänzend | glatt glänzend |
| nach 8 Wochen 20°C | glatt, glänzend | glatt glänzend |
| nach 8 Wochen 40°C | glatt, glänzend | glatt glänzend |
| Viskosität (20°C)[2] | | |
| nach 1 Tag | 66 Pa.s | 37 Pa.s |
| nach 8 Wochen | 77 Pa.s | 43 Pa.s |

[1] Kolloiddisperses Gemisch von Cetyl/stearylalkohol, Natrium-cetyl/stearylsulfat und nichtionogenen Emulgatoren.
[2] Die Messung der Viskosität erfolgt mit einem Brookfield-Rotationsviskosimeter.

3.2 Sportcreme (W/O-Emulsion)

| | | |
|---|---|---|
| Wollwachs | 5 | — |
| Wollwachs-Ersatzprodukt (nach 2.) | — | 5 |
| W/O-Grundlage (Protegin$^{(R)}$)[3] | 20 | 20 |
| Mikrowachs | 2,5 | 2,5 |
| Vaseline (weiß) | 2,5 | 2,5 |
| Paraffinöl | 2,5 | 2,5 |
| Mg $SO_4 \cdot 7\ H_2O$ | 0,4 | 0,4 |
| Glycerin | 2,0 | 2,0 |
| Wasser | 65,1 | 65,1 |
| Emulgierbarkeit | gut | gut |
| Struktur, Aussehen nach Herstellung | glatt, glänzend | glatt, glänzend |
| nach 8 Wochen bei 20°C | glatt, glänzend | glatt, glänzend |
| nach 8 Wochen bei 40°C | glatt, glänzend | leicht grießig |
| Viskosität (20°C) | | |
| nach 1 Tag | 225 Pa.s | 600 Pa.s |
| nach 8 Wochen | 200 Pa.s | 450 Pa.s |

[3] Mischung verschiedener nichtionogener Emulgatoren, Sterinen, aliphatischen Alkoholen, Fettsäure-estern, Wachsen und Paraffinkohlenwasserstoffen; Hersteller: Goldschmidt AG, Essen

### 3.3 Stearatcreme (O/W-Emulsion)

| | | |
|---|---|---|
| Wollwachs | 2 | — |
| Wollwachs-Ersatzprodukt (nach 2.) | — | 2 |
| Stearin (50% $C_{16}$, 50% $C_{18}$) | 14 | 14 |
| Cetylalkohol | 3 | 3 |
| Paraffinöl | 1 | 1 |
| Glycerin | 2 | 2 |
| Aminomethylpropandiol | 1 | 1 |
| Wasser | 77 | 77 |
| Emulgierbarkeit | gut | gut |
| Struktur, Aussehen nach Herstellung | leicht gelb, glänzend | glatt, glänzend |
| nach 8 Wochen | leicht inhomogen | leicht inhomogen |
| Viskosität (20°C) | | |
| nach 1 Tag | 650 Pa.s | 275 Pa.s |
| nach 8 Wochen | 487 Pa.s | 287 Pa.s |

### 3.4 Tagescreme (O/W-Emulsion)

| | | |
|---|---|---|
| Wollwachs | 2 | — |
| Wollwachs-Ersatzprodukt (nach 2.) | — | 2 |
| O/W-Cremegrundlage (Te gin[(R)][4]) | 12 | 12 |
| Cetylalkohol | 1 | 1 |
| Olivenöl | 3 | 3 |
| Glycerin | 5 | 5 |
| Wasser | 77 | 77 |
| Emulgierbarkeit | gut | gut |
| Struktur, Aussehen nach Herstellung | glatt, glänzend | glatt, glänzend |
| nach 8 Wochen 20°C | glatt, glänzend | glatt, glänzend |
| nach 8 Wochen 40°C | glatt, glänzend | leicht inhomogen |
| Viskosität (20°C) | | |
| nach 1 Tag | 125 Pa.s | 125 Pa.s |
| nach 8 Wochen | 157 Pa.s | 150 Pa.s |

[4] Glycerin-mono-distearat, Hersteller: Goldschmidt AG, Essen

### 3.5 Hautcreme, wasserfrei

| | | |
|---|---|---|
| Wollwachs | 10 | — |
| Wollwachs-Ersatzprodukt (nach 2.) | — | 10 |
| Cetylalkohol | 10 | 10 |
| Decyloleat (Cetiol V[R]) | 25 | 25 |
| Vaseline, weiß | 35 | 35 |
| Hartparaffin (52°C) | 5 | 5 |
| Bienenwachs, weiß | 15 | 15 |
| Struktur und Aussehen nach Herstellung | glatt u. fest | glatt u. fest |
| nach 8 Wochen 20°C | glatt u. fest | glatt u. fest |
| nach 8 Wochen 40°C | glatt u. fest | glatt u. fest |

### 3.6 Nährcreme (O/W-Emulsion)

| | | |
|---|---|---|
| Wollwachs | 3 | — |
| Wollwachs-Ersatzprodukt (nach 2.) | — | 3 |
| Cetylalkohol | 1,5 | 1,5 |
| Stearin (50% $C_{16}$, 50% $C_{18}$) | 12 | 12 |
| 2-Octyl-dodecanol (Eutanol G[R]) | 12 | 12 |
| Capoyl-caprinsäure-triglycerid (Myritol 318[R] | 6 | 6 |
| Bienenwachs, weiß | 2 | 2 |
| Paraffinöl, dickfl. | 5 | 5 |
| Glycerin | 6 | 6 |
| Triethanolamin | 1,5 | 1,5 |
| Wasser | 51,0 | 51,0 |
| Emulgierbarkeit | gut | gut |
| Struktur und Aussehen nach Herstellung | glatt, glänzend | glatt, glänzend |
| nach 8 Wochen 20°C | leicht inhomogen | leight inhomogen |
| nach 8 Wochen 40°C | glatt, stabil | glatt, stabil |
| Viskosität (20°C) | | |
| nach 1 Tag | 275 Pa.s | 425 Pa.s |
| nach 8 Wochen | 462 Pa.s | 412 Pa.s |

3.7 Babycreme (O/W-Emulsion)

| | | |
|---|---|---|
| Wollwachs | 10 | — |
| Wollwachs-Ersatzprodukt (nach 2.) | — | 10 |
| Palmitin/-stearinsäure-mono/diglycerid (Cutina MD[R]) | 16 | 16 |
| Cetyl/stearylalkoholpoly(12 EO) glycolether | 3 | 3 |
| Decyloleat (Cetiol V[R]) | 10 | 10 |
| Capoyl/-caprinsäure-triglycerid (Myritol 318[R]) | 4 | 4 |
| Calendulaöl | 3 | 3 |
| Wasser | 54 | 54 |
| Emulgierbarkeit | gut | gut |
| Struktur und Aussehen nach Herstellung | glatt, leicht matt | glatt, leicht matt |
| nach 8 Wochen 20°C | glatt, leicht matt | leicht inhomogen |
| nach 8 Wochen 40°C | leicht inhomogen | leicht inhomogen |
| Viskosität (20°C) | | |
| nach 1 Tag | 350 Pa.s | 437 Pa.s |
| nach 8 Wochen | 387 Pa.s | 437 Pa.s |

3.8 Babycreme (W/O-Emulsion)

| | | |
|---|---|---|
| Wollwachs | 5 | — |
| Wollwachs-Ersatzprodukt (nach 2.) | — | 5 |
| W/O-Emulgator (Dehymuls E[R]) | 7 | 7 |
| Decyloleat (Cetiol V[R]) | 10 | 10 |
| Vaseline, weiß | 15 | 15 |
| Talkum | 15 | 15 |
| Zinkoxid | 10 | 10 |
| Wasser | 38 | 38 |
| Emulgierbarkeit | gut | gut |
| Struktur und Aussehen nach Herstellung | weich, glänzend | weich, glänzend |
| nach 8 Wochen 20°C | weich, glänzend | weich, glänzend |
| nach 8 Wochen 40°C | weich, glänzend | weich, glänzend |
| Viskosität (20°C) | | |
| nach 1 Tag | 400 Pa.s | 725 Pa.s |
| nach 8 Wochen | 675 Pa.s | 737 Pa.s |

Die in den Rezepturen verwendeten Produkte

Emulgade F[R]    Myritol 318[R]

Cetiol V[R]    Cutina MD[R]

Eutanol G[R]    Dehymuls E[R]

werden hergestellt und geliefert durch Fa. Henkel KGaA, Düsseldorf.

**Patentansprüche**

1. Wollwachs-Ersazprodukt auf Basis synthetischer grenzflächenaktiver Fettfolgeprodukte, dadurch gekennzeichnet, daß es aus

40—60 Gew.-% eines Mischesters aus äquimolaren Mengen eines Pentaerythrit-di-fettsäureesters und eines Zitronensäure-di-fettalkoholesters,
20—45 Gew.-% Ölsäure-mono-diglycerid,
3—10 Gew.-% Palmitin- und/oder Stearinsäuremono-diglycerid und
3—10 Gew.-% eines Anlagerungsproduktes von 3—7 Mol Ethylenoxid an ein pflanzliches Sterin besteht, wobei die Summe der Bestandteile jeweils 100 Gew.-% betragen muß.

2. Wollfett-Ersatzprodukt nach Anspruch 1, dadurch gekennzeichnet, daß es aus

40—60 Gew.-% eines Mischesters aus äquimolaren Mengen eines Pentaerythrit-di-kokosfettsäureesters und eines Zitronensäure-di-octadecylesters,
25—45 Gew.-% Ölsäure-mong-diglycerid,
3—10 Gew.-% Palmitin- und/oder Stearinsäure-mono-diglycerid
3—10 Gew.-% eines Anlagerungsproduktes von ca. 5 Mol Ethylenoxid an ein raffiniertes Sojasterin besteht.

**0 093 341**

3. Wollwachs-Ersatzprodukt nach Anspruch 1 und 2, dadurch gekennzeichnet, daß es aus

50 Gew.-% des Mischesters aus äquimolaren Mengen von Pentaerythrit-di-kokosfettsäureester und Zitronensäure-di-n-octadecylester,
40 Gew.-% Ölsäure-mono-diglycerid
5 Gew.-% Palmitin und/oder Stearinsäure-mono-diglycerid
5 Gew.-% eines Anlagerungsproduktes von 5 Mol Ethylenoxid an raffiniertes Sojasterin

besteht.
4. Verwendung eines Wollwachs-Ersatzproduktes gemäß Anspruch 1—3 zur Herstellung kosmetischer und pharmazeutischer Salben, Cremes, Emulsionen und Stiftpräparate.

**Revendications**

1. Produit de remplacement de la cire de suint à base de dérivés gras tensio-actifs synthétiques, caractérisé en ce qu'il comprend:

40—60% en poids d'un ester mixte de quantités équimolaires d'un diester d'acide gras de pentaérythrite et d'un diester d'alcool gras d'acide citrique,
20—45% en poids de mono-diglycéride d'acide oléique,
3—10%. en poids de mono-diglycéride d'acide palmitique et/ou stéarique, et
3—10% en poids d'un produit de fixation de 3—7 moles d'oxyde d'éthylène sur une stérine végétale, la somme des constituants devant représenter chaque fois 100% en poids.

2. Produit de remplacement de la cire de suint selon la revendication 1, caractérisé en ce qu'il comprend:

40—60% en poids d'un ester mixte de quantités équimolaires d'un diester d'acide gras de coco de pentaérythrite et d'un diester octadécylique d'acide citrique,
25—45% en poids de mono-diglycéride d'acide oléique,
3—10% en poids de mono-diglycéride d'acide palmitique et/ou stéarique,
3—10% en poids d'un produit de fixation d'environ 5 moles d'oxyde d'éthylène sur une stérine de soya raffinée.

3. Produit de remplacement de la cire de suint selon les revendications 1 et 2, caractérisé en ce qu'il comprend:

50% en poids de l'ester mixte de quantités équimolaires de diester d'acide gras de coco de pentaérythrite et de diester n-octadécylique d'acide citrique,
40% en poids de mono-diglycéride d'acide oléique,
5% en poids de mono-diglycéride d'acide palmitique et/ou stéarique,
5% en poids d'un produit de fixation de 5 moles d'oxyde d'éthylène sur de la stérine de soya raffinée.

4. Utilisation d'un produit de remplacement de la cire de suint selon les revendications 1 à 3 pour la fabrication de préparations en bâtons, d'émulsions, de crèmes et de pommades cosmétiques et pharmaceutiques.

**Claims**

1. A wool wax substitute based on synthetic surface-active fat derivatives, characterized in that it consists of

40—60% by weight of a mixed ester of equimolar quantities of a pentaerythritol difatty acid ester and a citric acid difatty alcohol ester,
20—45% by weight oleic acid mono-diglyceride,
3—10% by weight palmitic acid and/or stearic acid mono-diglyceride and
3—10% by weight of an adduct of 3—7 moles ethylene oxide with a vegetable sterol, the sum of the constituents having to be 100% by weight.

2. A wool fat substitute as claimed in Claim 1, characterized in that it consists of

40—60% by weight of a mixed ester of equimolar quantities of a pentaerythritol dicoconut oil fatty acid ester and a citric acid di-octadecyl ester,
25—45% by weight oleic acid mono-diglyceride,
3—10% by weight palmitic acid and/or stearic acid mono-diglyceride,
3—10% by weight of an adduct of ca. 5 moles ethylene oxide with a refined soya sterol.

11

3. A wool wax substitute as claimed in Claims 1 and 2, characterized in that it consists of

50% by weight of the mixed ester of equimolar quantities of pentaerythritol dicoconut oil fatty acid ester and citric acid di-n-octadecyl ester,
40% by weight oleic acid mono-diglycerids,
5% by weight palmitic and/or stearic acid mono-diglyceride,
5% by weight of an adduct of 5 moles ethylene oxide with refined soya sterol.

4. The use of the wool wax substitute claimed in Claims 1 to 3 for the production of cosmetic and pharmaceutical ointments, creams, emulsions and stick preparations.